# EUROPEAN PATENT SPECIFICATION

(11) **EP 1 584 629 B1**
(45) Date of publication and mention of the grant of the patent: **12.09.2007**
(21) Application number: 05102717.5
(22) Date of filing: 07.04.2005
(51) Int. Cl.: C07K 14/705, C12Q 1/68

(54) **An in vitro method for the diagnosis of neoplastic diseases by methylation analysis of the CDH4 gene, oligonucleotides and kits useful in such method**
In vitro Verfahren zur Diagnose von neoplastischen Erkrankungen durch Analyse des Methylierungsstatus des CDH4 Gens, Oligonukleotide und Kits zur Durchführung eines solchen Verfahrens
Méthode in vitro pour la détection de maladies neoplastiques par analyse de la methylation du gène CDH4, oligonucleotides et kits utiles pour une telle méthode

(30) Priority: 09.04.2004 IT TO20040224
(43) Date of publication of application: 12.10.2005
(73) Proprietor: Universita' Degli Studi di Ferrara, 44100 Ferrara (IT)
(72) Inventor: Miotto, Elena, 44021, CODIGORO (Ferrara) (IT); Negrini, Massimo, 44100, FERRARA (IT); Sabbioni, Silvia, 44100, FERRARA (IT)
(74) Representative: Comoglio, Elena

(56) References cited:
- TOYOOKA SHINICHI ET AL: "Aberrant methylation of the CDH13 (H-cadherin) promoter region in colorectal cancers and adenomas." CANCER RESEARCH. 15 JUN 2002, vol. 62, no. 12, 15 June 2002 (2002-06-15), pages 3382-3386, XP002336362 ISSN: 0008-5472
- MIOTTO ELENA ET AL: "Frequent aberrant methylation of the CDH4 gene promoter in human colorectal and gastric cancer." CANCER RESEARCH. 15 NOV 2004, vol. 64, no. 22, 15 November 2004 (2004-11-15), pages 8156-8159, XP002336363 ISSN: 0008-5472

## Description

### Field of the invention

The present invention relates to an *in vitro* method for the diagnosis of solid tumours and haematological neoplastic diseases in humans, to nucleic acids and oligonucleotides useful in said method and to a kit comprising said oligonucleotides.

### Background of the invention

It is known that various human diseases, in particular neoplastic diseases and some haematological disorders such as for example leukaemia, are characterised by altered functionality of specific genes involved in the normal physiology of the cell. These genes have been divided into two conceptual categories, namely oncogenes and oncosuppressor genes, depending on whether they are activated or inactivated during the neoplastic transformation process. One of the main mechanisms for inactivating the oncosuppressor genes described in the scientific literature is hypermethylation of the promoter.

DNA methylation, namely the bond of the methyl group (-CH₃) to cytosine present within the context of the CpG dinucleotide (C = cytosine; G = guanine; p = monophosphate), is one of the commonest and best known epigenetic modifications which DNA may undergo in mammalian cells.

DNA methylation is known to have a regulatory function, in particular of gene transcription. It has in fact been observed that the gene promoters often contain regions which are particularly rich in CpG dinucleotides, known as *CpG islands,* the cytosines of which are generally unmethylated when the gene is transcriptionally active. However, when the gene's transcriptional ability is negatively regulated (i.e. when the gene is inactivated), the cytosines of the CpG islands present in the gene promoters are methylated. DNA methylation, also by means of the hypoacetylation of the histones, serves to compact the chromatin, so preventing access by the transcription factors necessary for gene transcription. It has also been reported that the methylation pattern is inherited by the cells derived from the cells in which the methylation process had originally taken place.

These factors gave rise to the idea that analysing the methylation status of the specific genes involved in the neoplastic transformation process could be a useful tool for the diagnosis of neoplastic diseases in humans. Both the scientific and patent literature have in fact reported numerous studies into the methylation status of genes involved in the development of human tumours.

However, a need still remains to identify new markers for neoplastic diseases which, on the one hand, make it possible to expand the knowledge currently available about the molecular mechanisms underlying carcinogenesis and, on the other hand, help to provide new methods both for the diagnosis and for the therapeutic treatment of human tumours.

### Brief description of the drawings

### Figure 1

Diagram of the region at the 5' end of the CDH4 gene, corresponding to Genbank sequence AL365229 (between 46200 and 50000). The diagram shows exon I and II of the CDH4 gene and the CpG dinucleotides are indicated by means of vertical bars. The density *(CpG island)* of these dinucleotides around the first exon of the CDH4 gene may be observed. The segments subjected to PCR amplification by primers cdh4-MF/MR (product A), cdh4-M3F/MR (product A nest) and cdh4-M2F/M3R (product B) are also indicated.

### Figure 2

Inverse relationship between methylation of the CDH4 promoter and expression of CDH4 mRNA.
A. DNA analysis of various human cell lines by means of MSP with CDH4-MR/CDH4-MR primers: the appearance of an amplification band is indicative of methylation of the region.
B. The same cell lines are analysed by means of RT-PCR to reveal CDH4 transcription. It may be noted that DNA methylation is associated with the absence of mRNA expression.

### Figure 3

Reexpression of the RNA of the CDH4 gene following demethylation.
A. Presence of methylation of the CDH4 promoter in the cell lines HeLa and SiHa revealed by means of MSP with cdh4_MF/cdh4_MR primers.
B. Demethylation of the DNA of the CDH4 promoter region in cell line HeLa following treatment with Aza-C, revealed by means of MSP with cdh4_UF/cdh4_UR primers.
C. Reexpression of the CDH4 gene in the samples treated with Aza-C, revealed by means of RT-PCR with cdh4_249F/cdh4_547R primers.

### Description of the invention

The present inventors have found that analysis of the methylation status of the human CDH4 gene, which codes for cadherin-4, is a useful tool for the diagnosis of various types of human neoplastic diseases, comprising solid tumours and haematological neoplastic diseases.

The possible role of the CDH4 gene in tumour metastasis has already been suggested in international patent application WO 01/77376. However, this patent application focuses narrowly on the analysis of the methylation pattern of the CD22 gene and provides no specific indication of the fact that altered methylation of the CDH4 gene could actually be correlated with neoplastic status, nor does it identify specific sequences of the CDH4 gene which are useful for diagnostic purposes.

After extensive research, the present inventors have found that the CpG island located at the 5' end of the CDH4 gene exhibits a high frequency of methylation in cells derived from human neoplasms, while being unmethylated in normal controls. The methylation status of said CpG island, which extends between nucleotide 46801 and nucleotide 49600 of Genbank sequence AL365229, is thus a useful marker of solid tumours and haematological neoplastic diseases in humans.

Accordingly, the present invention provides a method for the diagnosis of solid tumours and haematological neoplastic diseases, characterised in that it comprises, in a sample of genomic DNA, the step of analysing the methylation status of the nucleotide sequence of the 5' region of the CDH4 gene between the nucleotide positions corresponding to nucleotides 46801 and 49600 of Genbank sequence AL365229 (SEQ ID NO: 18), methylation of said sequence being indicative of neoplastic disease.

In the present description, the phrase "nucleotide positions corresponding to nucleotides 46801 and 49600 of Genbank sequence AL365229" denotes the fact that the nucleotide positions of the CDH4 gene of the genomic DNA to be analysed are numbered on the basis of the numbering of Genbank sequence AL365229.

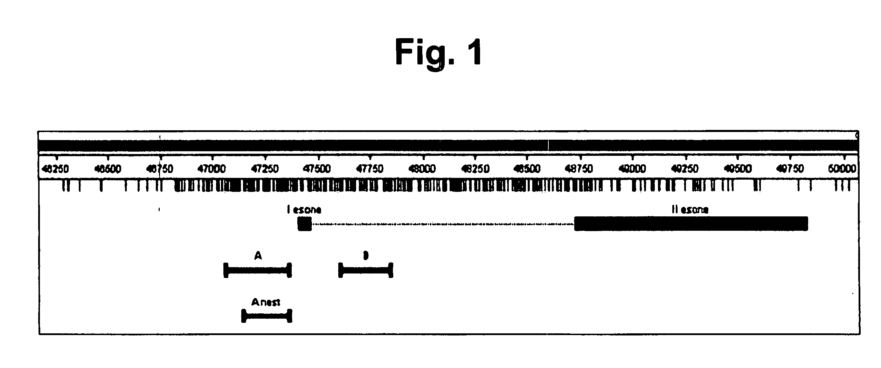

As shown in Figure 1, the CpG island at the 5' end of the CDH4 gene covers a region of approximately 3 kb which includes the 5' region, the first exon and extends into the first intron of the gene.

As will be described in greater detail below, the inventors have also found that the 5' region of the CpG island, namely the region between the nucleotide positions corresponding to nucleotides 47050-47376 of Genbank sequence AL365229, performs the function of a promoter and that aberrant methylation of this region, apart from being a marker of tumorous disorders, also correlates with inhibition of gene transcription. This result clarifies the molecular mechanism of the involvement of the CDH4 gene in the neoplastic process.

In a preferred embodiment, the diagnostic method of the invention thus comprises analysis of the methylation status of the nucleotide sequence of the CDH4 gene between the nucleotide positions corresponding to nucleotides 47050 and 47376 of Genbank sequence AL365229 (SEQ ID NO.: 19), methylation of said sequence being indicative of neoplastic disease.

The methylation status of the CpG island may be analysed using any suitable process, for example by using methods which include chemical treatment of the DNA with a reagent capable of converting methylation differences into sequence differences. Preferably, a chemical reagent is used to this end which is capable of converting the unmethylated cytosine bases into uracil or any other base capable of pairing with a base other than guanine.

One reagent which is particularly preferred for this purpose is bisulfite, preferably sodium bisulfite, in combination with hydroquinone. In this chemical reaction, the unmethylated cytosines present in the DNA are in fact deaminated to uracil which, in terms of base pairing, corresponds to thymidine. The methylated cytosines, on the other hand, are resistant to the chemical treatment and thus do not undergo any modification. Consequently, the methylcytosines present in the original genomic DNA, which could not initially be distinguished from the cytosines because they exhibited the same pairing characteristics, remain the only cytosines present in the DNA after the chemical treatment and may then be identified using standard molecular biology methods.

The base sequence of the genomic DNA which has been chemically treated as illustrated above may, for example, be analysed by direct sequencing.

Alternatively, it is possible to use methods such as Methylation-Specific PCR (MSP), based on the methylation-specific amplification of segments of the CpG island of the sample of chemically treated genomic DNA.

Such methods make use of oligonucleotides which are capable of undergoing selective hybridisation with the modified sequences of the methylated or unmethylated CpG island obtained by means of the above-described chemical treatment.

Chemically treating a sample of genomic DNA including the CpG island of the CDH4 gene (nucleotides 46801-49600 of Genbank sequence AL365229) as described above in fact gives rise to two modified strands, each of which is derived from one of the two strands of the treated genomic DNA, which are, however, no longer complementary to one another.

The sequences designated SEQ ID NO: 1 and SEQ ID NO: 2 are the nucleotide sequences of the two modified strands which are obtained when all the dinucleotides of the original CpG island of the CDH4 gene are methylated.

The sequences designated SEQ ID NO: 3 and SEQ ID NO: 4 are the nucleotide sequences of the two modified strands which are obtained when none of the dinucleotides of the original CpG island of the CDH4 gene is methylated.

Sequences SEQ ID NO: 1, SEQ ID NO: 2, SEQ ID NO: 3 and SEQ ID NO: 4 were determined on the basis of Genbank sequence AL365229 (nucleotides 46801-49600) of the CDH4 gene.

Oligonucleotides at least 10 nucleotides in length which are complementary or identical to a segment of a target sequence selected from the group consisting of SEQ ID NO: 1, SEQ ID NO: 2, SEQ ID NO: 3, SEQ ID NO: 4, are a useful tool for analysing the methylation status of the CpG island of a sample of genomic DNA, since the hybridisation thereof depends on the methylation status of the original CpG island of the sample. Such oligonucleotides fall within the scope of the present invention. Preferably, the oligonucleotides of the invention are complementary or identical to a segment of a target sequence as defined above comprising at least one CpG dinucleotide.

The above-defined oligonucleotides may be used in the diagnostic process of the invention as amplification primers or as hybridisation probes. In the latter case, they preferably have a length of at least 18 nucleotides.

In a preferred embodiment, the oligonucleotides of the invention have been designed on the basis of the nucleotide sequences between positions 250 and 576 of SEQ ID NO: 1 and SEQ ID NO: 3, and between nucleotide positions 2225 to 2551 of SEQ ID NO: 2 and SEQ ID NO: 4. Said nucleotide positions correspond to the promoter of the CDH4 gene, namely to nucleotide positions 47050-47376 of Genbank sequence AL365229. The nucleotide sequences between positions 250 and 576 of SEQ ID NO: 1 and of SEQ ID NO: 3 are respectively designated as SEQ ID NO: 5 and SEQ ID NO: 7.

The nucleotide sequences between positions 2225 to 2551 of SEQ ID NO: 2 and of SEQ ID NO: 4 are respectively designated as SEQ ID NO: 6 and SEQ ID NO: 8.

The diagnostic method of the invention using the above-described oligonucleotides may, for example, be a detection method based on DNA amplification, optionally followed by detection of the amplified DNA fragments by means of a hybridisation probe.

In such a method, a sample of genomic DNA to be investigated is chemically pretreated with a reagent capable of converting the unmethylated cytosine bases into uracil or any other base capable of pairing with a base other than guanine.

The sample of genomic DNA to be analysed is preferably obtained from a source such as blood, serum, plasma, bronchial washings, expectoration, saliva, intestinal washings, urine, faeces, ejaculate, pads of various origins, needle aspirates, lymph node biopsies, or combinations thereof.

The above-stated chemical pretreatment of the genomic DNA is preferably performed with sodium bisulfite and hydroquinone.

Following chemical pretreatment, two modified strands, which are, however, no longer complementary to one another, are obtained from the original double-stranded genomic DNA.

After the chemical pretreatment step, one of the modified strands obtained by pretreatment of the genomic DNA may be subjected to an amplification reaction using as the amplification primers at least two oligonucleotides at least 10 nucleotides in length, wherein one of said oligonucleotides is complementary to a first segment of a target sequence selected from the group consisting of SEQ ID NO: 1, SEQ ID NO: 2, SEQ ID NO: 5 and SEQ ID NO: 6 and the other oligonucleotide is identical to a second segment of said target sequence, the second segment of the target sequence being downstream from the first segment. According to one embodiment, at least one of said first and second segments of the target sequence comprises at least one CpG dinucleotide. In this case, the amplification reaction is methylation-specific, since it uses a designated pair of oligonucleotide primers to amplify specifically a modified strand derived from the sequence of the original methylated CpG island. In this embodiment, detection of the amplified DNA fragments, if present, may be performed by any per se known method, for example by electrophoresis on agarose gel and staining with a DNA intercalating agent, such as for example ethidium bromide.

According to another embodiment, neither the first nor the second segment of the target sequence comprises a CpG dinucleotide. In this case, the amplification reaction is not methylation-specific. Consequently, detection of the amplified fragments, if present, will be performed by hybridisation with a methylation-specific probe, namely an oligonucleotide at least 18 nucleotides in length which is complementary to a third segment of the target sequence of the amplification primers, said third segment being located between the said first and second segments of the target sequence and comprising at least one CpG dinucleotide. The third segment of the target sequence may optionally be partially overlapped to the first or the second segment.

The hybridisation probe is preferably labelled with a detectable marker, for example a radioisotope, a fluorescent molecule, a chemiluminescent molecule, an enzyme capable of reacting with a chromogenic substrate, or with an avidin/biotin or antigen/antibody system which is itself labelled.

In both the above-described embodiments of the method, the amplification reaction is preferably a polymerase chain reaction (PCR). Still more preferred is an amplification reaction in two steps which comprises a primary PCR followed by a semi-nested or nested secondary PCR.

Since the modified genomic DNA representing the target of the amplification reaction is initially single-stranded, in the first PCR cycle only one of the two primers will initially hybridise with the target strand. The first hybridised primer will be extended by a polymerase using the target strand as template. Subsequently, the second primer will hybridise with the newly synthesised chain and will be extended by the polymerase using the newly synthesised chain as the template.

In this manner, the initial single-stranded target DNA will be converted into double-stranded DNA. In the subsequent cycles of the PCR reaction, in contrast, the two amplification primers will each simultaneously hybridise with one strand of the double-stranded target DNA.

Table 1 shows some specific examples of oligonucleotides suitable as primers or probes in the diagnostic method of the invention.

**Table 1.**

| PRIMER | SEQUENCE | SEQ ID no. |
|---|---|---|
| Cdh4_MF | CGTTTTTAAGGTTCGCGTCG | 9 |
| Cdh4_M2F | TCGGCGTTAACGCGTTCGGC | 10 |
| Cdh4_M3F | TTTGTAGTTTCGAGCGCGC | 11 |
| Cdh4_MR | GAAAACCCGCTCCCTACC | 12 |
| Cdh4_M2R | ATAAAAACGACCTCGCGACGCGC | 13 |
| Cdh4_UF | GTGTTTTTAAGGTTTGTGTTGG | 14 |
| Cdh4_UR | AAAAACCCACTCCCTACC | 15 |

The oligonucleotides cdh4_MF, cdh4_MR and cdh4_M3F were designed to specifically hybridise with SEQ ID NO: 5. When used as the primers in a PCR amplification, the pair of oligonucleotides cdh4_MF and cdh4_MR amplifies a sequence of 326 bp between nucleotide 1 and nucleotide 326 of SEQ ID NO: 5; the pair of oligonucleotides cdh4_M3F and cdh4_MR amplifies a region of 245 bp between nucleotide 82 and nucleotide 326 of SEQ ID NO: 5.

The oligonucleotides cdh4_UF and cdh4_UR were designed to hybridise with SEQ ID NO: 7. When used as the primers in a PCR amplification, the pair of oligonucleotides cdh4_UF and cdh4_UR amplifies a sequence of 327 bp between nucleotide 1 and nucleotide 327 of SEQ ID NO: 7.

Oligonucleotides in the region of the CpG island located in the first intron have also been designed. These oligonucleotides are designed to hybridise with the portion of SEQ ID NO: 1 between nucleotide 799 (cdh4_M2F) and nucleotide 1061 (cdh4_M2R).

A kit for performing the diagnostic method of the invention also falls within the scope of the present invention, which kit may comprise for example a pair of amplification primers as defined above optionally in combination with a reagent capable of converting the unmethylated cytosine bases into uracil or any other base capable of pairing with a base other than guanine, for example sodium bisulfite in combination with hydroquinone. The kit may further comprise DNA detection means, such as for example a methylation-specific hybridisation probe as defined above, and/or DNA amplification means, for example a polymerase enzyme. As an alternative to the probe, the kit may comprise a DNA detection reagent such as a DNA intercalating agent, for example ethidium bromide. Finally, the kit of the invention may comprise a second pair of amplification primers at least 10 nucleotides in length, one of said oligonucleotides being complementary to a first segment of a target sequence selected from the group consisting of SEQ ID NO: 3, SEQ ID NO: 4, SEQ ID NO: 7 and SEQ ID NO: 8 and the other being identical to a second segment of said target sequence, the second segment being downstream from the first segment. Preferably, at least one of said first and second segments of the target sequence comprises at least one TpG dinucleotide. The second pair of primers is used as a control that the DNA actually has been modified following treatment with bisulfite, so making it possible to validate a negative result of amplification with primers designed on the basis of the modified nucleotide sequences derived from the methylated CpG island.

The following Examples are provided by way of illustration and are not intended to limit the scope of the invention as defined in the appended claims.

### EXAMPLES

### Example 1: analysis of the methylation status of the CDH4 gene in tumour cell lines

The methylation status of the CpG island of the CDH4 gene was analysed in cell lines Hela, SiHa, MCF-7, SkLu-1, BT20, RKO, Hey, G401 and 293. Cell line MDA-MB-231, in which CDH4 is expressed, was used as a negative methylation control. These cell lines were also analysed by CDH4 gene transcription analysis, using line MDA-MB-231 as a positive transcription control.

Methylation status was analysed using the MSP method with chemical treatment of the genomic DNA by sodium bisulfite and hydroquinone and PCR amplification using the cdh4_MF/cdh4_MR primer pair.

Transcription was analysed by reverse transcription of the messenger RNA into cDNA, followed by PCR using the following primers:
cdh4_249F: GTTGGGGCAGATGGGACAGT (SEQ ID NO: 16)
cdh4_547R: ACGTTGATGGGCGGGATGAC (SEQ ID NO: 17)

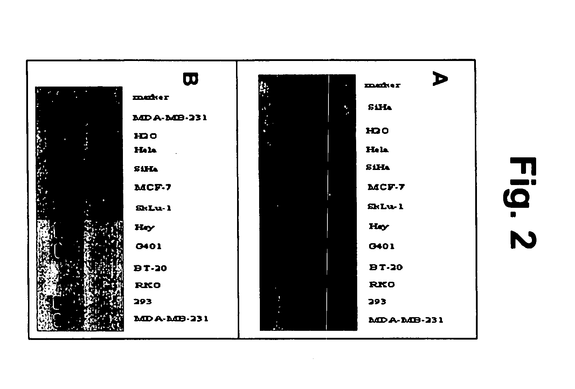

The results obtained are shown respectively in panel A (methylation) and panel B (transcription) of Figure 2. Figure 2 shows that the CpG island at 5' of the CDH4 gene is methylated in the lines Hela, SiHa, MCF-7, SkLu-1, BT20 and RKO and that the CDH4 gene is not expressed in these lines, while in lines Hey, G401 and 293, the CpG island is not methylated and the CDH4 gene is expressed.

This investigation thus made it possible to establish that there is a direct relationship between methylation and inhibition of transcription in the CDH4 gene.

Studies carried out on cell line MDA-MB-231 also made it possible to identify a specific region of the CpG island which proved to be specifically involved in regulating transcription of the CDH4 gene. This region which regulates transcription is located at 5' of the first exon. In cell line MDA-MB-231, in which CDH4 is normally transcribed, methylation has in fact been observed in the intron (which extends from nucleotide 47464 to 49831 of Genbank sequence AL365229), but absence of methylation at 5' of the gene (nucleotides 47050-47376 of the same sequence). Methylation of the CpG region between the nucleotides corresponding to positions 47050-47376 of Genbank AL365229 thus inhibits functioning of the CDH4 gene promoter.

### Example 2: Effect of treatment with a demethylating drug

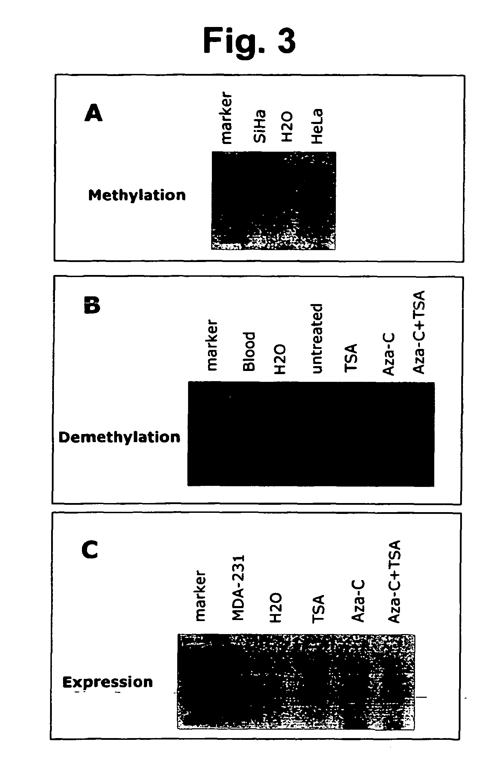

It has been demonstrated in another investigation that demethylation of the CpG island brought about by treatment with a demethylating drug reestablishes expression of the CDH4 gene in cell line HeLa. The results of this experiment are shown in Figure 3.

Cell line HeLa was subjected to methylation analysis by the MSP technique using the primers cdh4_MF/cdh4_MR. As expected, the promoter of the CDH4 gene proved to be methylated in this cell line (see panel A of Figure 3).

Cell line HeLa was subsequently subjected to MSP using the cdh4_UF/cdh4_UR primer pair which is non-methylation-specific. DNA extracted from peripheral blood of healthy individuals was used as a positive control. The results obtained are shown in panel B of Figure 3. As expected, there is no DNA amplification in the HeLa cells (designated as "untreated" in Fig. 3B), while the positive control ("blood") is amplified.

The HeLa cells were then subjected to treatment with: (i) the demethylating drug 5-AZA-2-deoxycytidine (5-AZA-CdR, designated as "AZA-C" in Figure 3); (ii) the hypoacetylation agent trichostatin-A ("TSA"); and (iii) 5-AZA-CdR in combination with TSA. Treatment with the demethylating drug brought about demethylation of the CpG island, as revealed by positive amplification with the primers cdh4UF/UR (see panel B of Figure 3). In contrast, the hypoacetylation agent trichostatin-A had no observable effect.

Finally, panel C of Figure 3 shows the results of the analysis of the expression of the CDH4 gene in HeLa cells after treatment with 5-AZA-CdR alone, 5-AZA-CdR + TSA and TSA alone (the positive control was the cell line MDA-MB-231). As expected, the CDH4 gene proved to be expressed in the cells treated with 5-AZA-CdR alone and with 5-AZA-CdR + TSA, but not in the cells treated with TSA alone.

This result is particularly significant because it provides conclusive proof of the causal relationship between methylation of the promoter and transcription of the CDH4 gene.

In summary, on the basis of the data obtained from the cell line studies, it could be concluded that methylation of the CDH4 promoter is present in 6 out of 10 of the analysed cell lines and that said methylation is responsible for inhibition of transcription of the CDH4 gene. This is thus potentially of significance in determining the neoplastic characteristics of the cell lines under investigation.

### Example 3: study of the methylation of the CDH4 gene in human tumours

When studying human tumorigenesis, the data obtained from cell lines are indicative of a possible pathogenic mechanism, but direct proof can only be obtained from studies on primary tumours.

Accordingly, different kinds of human tissues, both neoplastic and non-neoplastic, have been studied for the presence of methylation of the CDH4 promoter.

The results indicate that the neoplastic tissues most frequently subject to methylation of the CDH4 promoter are gastric carcinoma (100% of cases) and colorectal carcinoma (78% of cases).

In order to rule out that the data obtained could be due to tissue-specific methylation or to methylation correlated to the age of the patients and thus not directly to the neoplastic nature of the tissue, samples of gastric and colorectal mucosa were also analysed from individuals not suffering from neoplastic disease. In neither case was any methylation found. These data demonstrate that methylation of the CpG island of the CDH4 gene arises during neoplastic transformation and may thus be used as a tumour-specific diagnostic marker. They also demonstrate a certain degree of specificity in the type of neoplasia in which methylation occurs.

It has additionally been evaluated whether methylation of the CDH4 gene occurs early or late in the neoplastic process. After carcinomas, colonic adenomas, i.e. benign neoplasms, were thus examined which, according to a well-known multiphase model of colorectal neoplasia, are an early stage of development towards the invasive carcinoma. Analysis of 5 colonic adenomas revealed them all to be positive in the CDH4 methylation test, so demonstrating that this epigenetic lesion occurs early.

17 samples of tissue adjacent to the colonic tumour and 21 samples of tissue adjacent to the stomach tumour, histopathological analysis of which had not detected alterations of a neoplastic type, were also analysed.

The CDH4 methylation test made it possible to identify the presence of CDH4 methylation in these samples too, so demonstrating that techniques based on epigenetic type markers have greater sensitivity, in comparison with conventional techniques, in detecting tissue changes which are of a probably pre-neoplastic nature.

Thanks to the high specificity and sensitivity of the analytical techniques, the alterations in methylation observed in human tumours may also be revealed in organic fluids. This phenomenon may be explained by the fact that tumour cells may become detached from the neoplastic mass and release DNA into the circulatory system or into the lumen of the organ from which they originate.

The attempt was thus made to detect the presence of circulating tumour DNA by analysing 11 samples of blood from patients with a colorectal tumour. The methylation test proved positive in 5 out of 11 samples (45%) by using simple PCR (50 cycles) while, under the same conditions, there were no traces of methylation in 17 samples of blood from healthy volunteers. When the test was performed by means of a primary PCR (cdh4-MF/cdh4-MR primers) followed by a semi-nested secondary PCR (cdh4-M3F/cdh4-MR primers), the rate of positives was increased to 9 out of 11 (82%) in samples of blood from patients suffering from colorectal carcinoma.

Table 2 below summarises all the above-mentioned results.

**Table 2. Methylation percentage of the CDH4 promoter in human tissues.**

| SAMPLE | | METHYLATION % |
|---|---|---|
| Stomach | Carcinoma | 100.0 (21/21) |
| | Mucosa adjacent to tumour | 81.0 (17/21) |
| Colon | Carcinoma | 77.6 (38/49) |
| | Mucosa adjacent to | 29.4 (5/17) |
| | tumour | |
| | Adenoma | 100.0 (5/5) |
| | Blood | 82.0 (9/11) |
| Breast | Carcinoma | 42.6 (20/47) |
| Cervix | Carcinoma | 35.0 (7/20) |
| Liver | Carcinoma | 4.3 (1/23) |
| Normal controls | Gastric mucosa | 0.0 (0/6) |
| | Colonic mucosa | 0.0 (0/3) |
| | Blood | 0.0 (0/17) |

These results indicate that the MSP technique applied to the CDH4 marker is sufficiently sensitive to detect traces of circulating tumour DNA and may thus be employed for diagnostic purposes by means of relatively simple assays which can be performed by non-invasive, low cost methods.

Besides blood, other organic fluids may also be analysed by the described methodology. Such fluids which may be mentioned by way of example are serum, plasma, bronchial washings, expectoration, saliva, intestinal washings, urine, faeces, ejaculate, pads of various origins, needle aspirates and lymph node biopsies.

In summary, the data obtained on primary tumours have made it possible to demonstrate the presence of the altered methylation of the CpG island of the CDH4 gene, in particular of the promoter region, with a frequency which varies depending on the type of neoplasia. In some cases, the frequency was 100% or very close to 100%, as in gastric and colorectal carcinomas. This alteration occurs early in the neoplastic process and may also be revealed by non-invasive investigations performed on biological samples of a non-neoplastic nature, such as for example blood.

### Example 4: materials and methods

Samples of tumour tissue were taken from patients with colorectal, gastric, hepatic, mammary, prostate, ovarian or cervical carcinoma during surgery and immediately frozen in liquid nitrogen.

The blood was collected from patients suffering from colorectal carcinoma before surgery. 3 ml of peripheral blood were collected in a Vacutainer tube containing 5.9 mg of EDTA.

The tumour tissue was lysed overnight at 37°C in Hirt's solution (50 mM Tris-HCl pH 8, 50 mM EDTA, 0.6% SDS) with the addition of 200 µg/ml of Proteinase K at 37°C overnight. The DNA was then extracted with phenol-chloroform, precipitated with 3 M Na acetate and ETOH and resuspended in TE (Tris-HCl pH 8 10 mM, EDTA 1 mM).

The DNA was extracted from the blood with PROMEGA kit cat. no. #A1120 starting from a volume of 600 µl of whole blood and resuspended in TE (Tris-HCl pH 8 10 mM, EDTA 1 mM).

The samples of DNA were chemically modified in accordance with the protocol described by Herman et al. (Proc. Natl. Acad. Sci. USA 93: 9821, 1996), to which some modifications were made. From each sample, 1 µg of DNA was diluted in 50 µl of H₂O and denatured with 3.5 µl of 3 M NaOH at 37°C for 10 minutes. 30 µl of 10 mM hydroquinone (Sigma cat. no. H-9003) and 520 µl of 3 M sodium bisulfite pH 5 (Sigma S-8890) were then added and the mixture incubated for 16 hours at 50°C. The samples were purified with 0.6 ml of resin from the DNA Wizard Cleanup kit (Promega cat. no. #A7280). Washing was performed 3 times with 80% isopropanol, on completion of which 100 µl of H₂O preheated to 70°C were added to resuspend the DNA. 11 µl of 3 M NaOH were added for 5 minutes at room temperature and the samples were precipitated with 66 µl of 10 M NH₄-acetate and ETOH using 1 µl of glycogen as the carrier (Invitrogen cat. no. #10814-010). Finally, the modified DNA was resuspended in 40 µl of H₂O preheated to 70°C.

PCR analysis on the samples of DNA from tumour tissue was performed with the primers cdh4 MF and cdh4 MR. PCR was performed with HotStart Taq DNA Polymerase (Qiagen cat. no. #203203) with the addition of 10% DMSO, under the following conditions:
95°C for 15 minutes;
95°C for 30 seconds, 60°C for 30 seconds, 72°C for 1 minute, 40 cycles;
72°C for 10 minutes.

PCR on the samples of DNA from blood was performed in two steps using in the first step (primary PCR) the cdh4_MF and cdh4_MR pair with 50 reaction cycles, and, in the second step (semi-nested secondary PCR), the primers cdh4_M3F and cdh4_MR with a further 50 reaction cycles, using 1 µl from the primary PCR reaction diluted 1:10.

The presence of amplificates was detected by subjecting the PCR products to electrophoresis in 1.5% agarose gel (Fisher cat. no. #AS-109) and revealed with ethidium bromide and ultraviolet light.

### Discussion

The results obtained indicate that aberrant methylation of the CpG island between nucleotide positions 46801 and 49600 of Genbank sequence AL365229, corresponding to the 5' region of the CDH4 gene, and in particular of the promoter region, is a useful diagnostic marker for neoplastic diseases. The possibility that the CDH4 gene has an oncosuppressor function would furthermore suggest that this type of investigation may be a possible tool for assessing the usefulness of using CDH4 as a tool for antineoplastic gene therapy.

The MSP methylation analysis technique (Methylation-Specific PCR) has been described by Herman et al., Proc. Natl. Acad. Sci. USA 93: 9821, 1996 and in US patent No. 5,786,146.

Of course, the diagnostic method of the invention may also be performed using other types of assays capable of revealing altered DNA methylation. Of these, the following may be mentioned by way of example: (i) "MethyLight", described by Eads et al., Cancer Research 59: 2302, 1999; (ii) Ms-SNuPE (Methylation-sensitive Single Nucleotide Primer Extension), described by Gonzalgo et al., Nucleic Acids Research 25: 2529, 1997; (iii) "COBRA" (Combined Bisulfite Restriction Analysis), described by Xiong et al., Nucleic Acids Research 25: 2532, 1997; "MS. AP-PCR" (Methylation-Sensitive Arbitrarily-Primed Polymerase Chain Reaction) described by Gonzalgo et al., Cancer Research 57: 594, 1997; "MCA" (Methylated CpG Island Amplification), described by Toyota et al., Cancer Research 59: 2307, 1999 and in patent WO 00/26401A1.

In the light of the frequency observed in gastrointestinal tract tumours, the CDH4 methylation marker is of particular significance in colorectal and gastric tumours. These neoplasias are among the most widespread in the Western population and, despite the progress made in biomedical research, mortality remains very high. This is primarily attributable to a poor prognosis as a result of the advanced stage of the disease at the time of diagnosis. Currently used diagnostic techniques are based on radiographic and/or endoscopic methods which are costly and invasive. An alternative to diagnostics by imaging is currently represented by testing for occult blood in the faeces or for tumour antigens in the serum. While the first test is somewhat unreliable because it is not entirely specific and is of low sensitivity, being incapable of diagnosing the presence of small neoplasms, tests based on detecting tumour antigens in serum are proving to be of little use since they are informative only once the disease reaches an advanced stage.

For this reason, it is desirable to identify markers of neoplasia which are characterised by a high level of specificity and are detectable at a very high level of sensitivity by means of largely uninvasive and low cost methods.

Aberrant methylation of the CpG island of CDH4 is a marker which meets these requirements.

As shown in the present study, methylation of the CpG island of CDH4 may be detected in various human tumours, in particular in gastric, colorectal and mammary carcinoma. However, the results obtained would suggest that this marker could be used in the diagnosis of a huge range of neoplastic diseases, including both solid tumours and neoplastic diseases of the blood, and in particular in the diagnosis of: primary tumours of the central nervous system, cerebral metastases, tumours of the eye, tumours of the pituitary gland, tumours of the thyroid gland, tumours of the adrenal cortex, tumours of the diffuse endocrine system, tumours of the head and neck, tumours of the lungs, malignant mesothelioma, thymomas and thymic tumours, tumours of the heart and major vessels, primary thoracic germ cell tumours, metastatic tumours of the thorax, oesophageal tumours, liver tumours, gastric tumours, tumours of the gallbladder and bile ducts, tumours of the exocrine pancreas, tumours of Vater's ampulla, tumours of the small intestine, tumours of the appendix and peritoneum, tumours of the colon and the rectum, tumours of the anus, renal tumours, tumours of the pelvis and the ureter, tumours of the bladder, tumours of the prostate gland, tumours of the penis and the urethra, testicular tumour, tumours of the vulva and the vagina, tumours of the cervix, endometrial cancer, Fallopian tube tumours, ovarian tumour, gynaecological sarcomas, mammary tumours, malignant melanoma, tumours of the skin, tumours of the bone, soft tissue sarcomas, Wilms' tumour, neuroblastoma, rhabdomyosarcoma, Kaposi's sarcoma, myelodysplastic syndrome, acute myeloid leukaemia of the adult and the child, chronic myeloid leukaemia, acute lymphocytic leukaemia of the adult, acute lymphoblastic leukaemia of the child, chronic lymphocytic leukaemia, hairy cell leukaemia, mast cell leukaemia, Hodgkin's disease, non-Hodgkin's lymphomas, fungoid mycosis and Sézary syndrome, plasma cell tumours, chronic myeloproliferative disorders (essential thrombocythaemia, myelofibrosis with myeloid metaplasia, true polycythaemia).

Methylation of this marker is furthermore specific to the neoplastic tissue, in that it was not found in normal samples. Furthermore, CDH4 methylation is also detectable in the blood of patients suffering from neoplasia, which is a considerable advantage in view to the development of non-invasive diagnostic assays performed on samples of biological material which are easy to take, such as serum, plasma, bronchial washings, expectoration, saliva, intestinal washings, urine, faeces, ejaculate, aspiration needle samples, pads or lymph nodes. Furthermore, since methylation of the CDH4 promoter occurs at an early stage of the disease, as exemplified by colonic adenomas, it is reasonable to suppose that this marker may be used for the early diagnosis of tumours. Given the potential oncosuppressor properties of the CDH4 gene (in other words a gene which is capable of inhibiting some essential characteristics of the neoplastic cell), determination of the methylation status of this gene may also be used to provide useful indications relating to the use of CDH4 in gene therapy protocols.

## Claims

1. A nucleic acid comprising a nucleotide sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7 and SEQ ID NO: 8.

2. An oligonucleotide at least 10 nucleotides in length, which is complementary or identical to a segment of a target sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7 and SEQ ID NO: 8.

3. The oligonucleotide according to claim 2, at least 18 nucleotides in length.

4. The oligonucleotide according to claim 2 or claim 3, wherein said target sequence is selected from the group consisting of SEQ ID NO: 1, SEQ ID NO: 2, SEQ ID NO: 5 and SEQ ID NO: 6, and said segment of the target sequence comprises at least one CpG dinucleotide.

5. The oligonucleotide according to claim 2 or claim 3, wherein said target sequence is selected from the group consisting of SEQ ID NO: 3, SEQ ID NO: 4, SEQ ID NO: 7 and SEQ ID NO: 8, and said segment of the target sequence comprises at least one TpG dinucleotide.

6. The oligonucleotide according to claim 1, selected from the group consisting of SEQ ID NO: 9, SEQ ID NO: 10, SEQ ID NO: 11, SEQ ID NO: 12, SEQ ID NO: 13, SEQ ID NO: 14 and SEQ ID NO: 15.

7. A method for the diagnosis of neoplastic disease comprising the step of analysing, in a sample of genomic DNA, the methylation status of the nucleotide sequence of the CDH4 gene between the nucleotide positions corresponding to nucleotides 46801 and 49600 of Genbank sequence AL365229 (SEQ ID NO: 18), methylation of said sequence being indicative of neoplastic disease.

8. The method according to claim 7, comprising the step of analysing, in a sample of genomic DNA, the methylation status of the nucleotide sequence of the CDH4 gene between the nucleotide positions corresponding to nucleotides 47050 and 47376 of Genbank sequence AL365229 (SEQ ID NO: 19), methylation of said sequence being indicative of neoplastic disease.

9. The method according to claim 7 or claim 8, comprising the steps of:
(i) chemically pretreating a sample of genomic DNA with a reagent capable of converting the unmethylated cytosine bases into uracil or any other base capable of pairing with a base other than guanine;
(ii) subjecting a strand of the pretreated genomic DNA to amplification using as amplification primers at least two oligonucleotides each at least 10 nucleotides in length, one of said oligonucleotides being complementary to a first segment of a target sequence selected from the group consisting of SEQ ID NO: 1, SEQ ID NO: 2, SEQ ID NO: 5 and SEQ ID NO: 6 and the other oligonucleotide being identical to a second segment of said target sequence, the second segment being downstream from the first segment, at least one of said first and second segments of the target sequence comprising at least one CpG dinucleotide; and
(iii) detecting the presence of amplified fragments of the pretreated genomic DNA, the presence of amplified fragments being indicative of neoplastic disease.

10. The method according to claim 7 or claim 8, comprising the steps of:
(i) chemically pretreating a sample of genomic DNA with a reagent capable of converting the unmethylated cytosine bases into uracil or any other base capable of pairing with a base other than guanine;
(ii) subjecting a strand of the pretreated genomic DNA to amplification using as amplification primers at least two oligonucleotides each at least 10 nucleotides in length, one of said oligonucleotides being complementary to a first segment of a target sequence selected from the group consisting of SEQ ID NO: 1, SEQ ID NO: 2, SEQ ID NO: 5 and SEQ ID NO: 6 and the other oligonucleotide being identical to a second segment of said target sequence, the second segment being downstream from the first segment; and
(iii) detecting the presence of amplified fragments of the pretreated genomic DNA by hybridisation with an oligonucleotide probe at least 18 nucleotides in length which is complementary to a third segment of said target sequence, said third segment being located between said first and said second segment and comprising at least one CpG dinucleotide, the presence of amplified fragments being indicative of neoplastic disease.

11. The method according to claim 10, wherein said probe is labelled with a detectable marker.

12. The method according to any of claims 9 to 11, wherein said amplification is a polymerase chain reaction (PCR).

13. The method according to claim 12, wherein said amplification is a primary PCR followed by a semi-nested or nested secondary PCR.

14. The method according to any of claims 9 to 13, wherein said reagent is bisulfite, preferably sodium bisulfite, in combination with hydroquinone.

15. The method according to any of claims 7 to 14, wherein said neoplastic disease is a solid tumour or a neoplastic disease of the blood.

16. The method according to claim 15, wherein said neoplastic disease is selected from the group consisting of primary tumours of the central nervous system, cerebral metastases, tumours of the eye, tumours of the pituitary gland, tumours of the thyroid gland, tumours of the adrenal cortex, tumours of the diffuse endocrine system, tumours of the head and neck, tumours of the lungs, malignant mesothelioma, thymomas and thymic tumours, tumours of the heart and major vessels, primary thoracic germ cell tumours, metastatic tumours of the thorax, oesophageal tumours, liver tumours, gastric tumours, tumours of the gallbladder and bile ducts, tumours of the exocrine pancreas, tumours of Vater's ampulla, tumours of the small intestine, tumours of the appendix and peritoneum, tumours of the colon and the rectum, tumours of the anus, renal tumours, tumours of the pelvis and the ureter, tumours of the bladder, tumours of the prostate gland, tumours of the penis and the urethra, testicular tumour, tumours of the vulva and the vagina, tumours of the cervix, endometrial cancer, Fallopian tube tumours, ovarian tumour, gynaecological sarcomas, mammary tumours, malignant melanoma, tumours of the skin, tumours of the bone, soft tissue sarcomas, Wilms' tumour, neuroblastoma, rhabdomyosarcoma, Kaposi's sarcoma, myelodysplastic syndrome, acute myeloid leukaemia of the adult and the child, chronic myeloid leukaemia, acute lymphocytic leukaemia of the adult, acute lymphoblastic leukaemia of the child, chronic lymphocytic leukaemia, hairy cell leukaemia, mast cell leukaemia, Hodgkin's disease, non-Hodgkin's lymphomas, fungoid mycosis and Sézary syndrome, plasma cell tumours, chronic myeloproliferative disorders.

17. The method according to claim 15, wherein said neoplastic disease is selected from the group consisting of gastric carcinoma, colorectal carcinoma and mammary carcinoma.

18. A kit for the diagnosis of neoplastic disease, comprising a pair of amplification primers as defined in claim 9 or claim 10.

19. The kit according to claim 18, additionally comprising a hybridisation probe as defined in claim 10.

20. The kit according to claim 18 or claim 19, additionally comprising a reagent capable of converting the unmethylated cytosine bases into uracil or any other base capable of pairing with a base other than guanine.

21. The kit according to any of claims 18 to 20, additionally comprising a second pair of amplification primers at least 10 nucleotides in length, one of said oligonucleotides being complementary to a first segment of a target sequence selected from the group consisting of SEQ ID NO: 3, SEQ ID NO: 4, SEQ ID NO: 7 and SEQ ID NO: 8 and the other being identical to a second segment of said target sequence, the second segment being downstream from the first segment.

22. The kit according to claim 21, wherein at least one of said first and second segments of the target sequence comprises at least one TpG dinucleotide.

23. The kit according to any of claims 18 to 22, additionally comprising DNA amplification means and/or DNA detection reagents.

## Patentansprüche

1. Nucleinsäure, umfassend eine Nucleotidsequenz, die ausgewählt ist aus der Gruppe, bestehend aus SEQ ID NO: 1, SEQ ID NO: 2, SEQ ID NO: 3, SEQ ID NO: 4, SEQ ID NO: 5, SEQ ID NO: 6, SEQ ID NO: 7 und SEQ ID NO: 8.

2. Oligonucleotid von mindestens 10 Nucleotiden Länge, das zu einem Segment einer Zielsequenz, die ausgewählt ist aus der Gruppe, bestehend aus SEQ ID NO: 1, SEQ ID NO: 2, SEQ ID NO: 3, SEQ ID NO: 4, SEQ ID NO: 5, SEQ ID NO: 6, SEQ ID NO: 7 und SEQ ID NO: 8, komplementär oder identisch ist.

3. Oligonucleotid gemäß Anspruch 2, das mindestens 18 Nucleotide lang ist.

4. Oligonucleotid gemäß Anspruch 2 oder Anspruch 3, wobei die Zielsequenz aus der Gruppe; bestehend aus SEQ ID NO: 1, SEQ ID NO: 2, SEQ ID NO: 5 und SEQ ID NO: 6, ausgewählt ist und das Segment der Zielsequenz mindestens ein CpG-Dinucleotid umfasst.

5. Oligonucleotid gemäß Anspruch 2 oder Anspruch 3, wobei die Zielsequenz aus der Gruppe, bestehend aus SEQ ID NO: 3, SEQ ID NO: 4, SEQ ID NO: 7 und SEQ ID NO: 8, ausgewählt ist und das Segment der Zielsequenz mindestens ein TpG-Dinucleotid umfasst.

6. Oligonucleotid gemäß Anspruch 1, ausgewählt aus der Gruppe, bestehend aus SEQ ID NO: 9, SEQ ID NO: 10, SEQ ID NO: 11, SEQ ID NO: 12, SEQ ID NO: 13, SEQ ID NO: 14 und SEQ ID NO: 15.

7. Verfahren zur Diagnose einer neoplastischen Erkrankung, umfassend den Schritt des Analysierens - in einer Probe von genomischer DNA - des Methylierungsstatus der Nucleotidsequenz des CDH4-Gens zwischen den Nucleotidpositionen, die den Nucleotiden 46801 und 49600 der Genbanksequenz AL365229 (SEQ ID NO: 18) entsprechen, wobei die Methylierung der Sequenz auf eine neoplastische Erkrankung hinweist.

8. Verfahren gemäß Anspruch 7, umfassend den Schritt des Analysierens - in einer Probe von genomischer DNA - des Methylierungsstatus der Nucleotidsequenz des CDH4-Gens zwischen den Nucleotidpositionen, die den Nucleotiden 47050 und 47376 der Genbanksequenz AL365229 (SEQ ID NO: 19) entsprechen, wobei die Methylierung der Sequenz auf eine neoplastische Erkrankung hinweist.

9. Verfahren gemäß Anspruch 7 oder Anspruch 8, umfassend die folgenden Schritte:
(i) das chemische Vorbehandeln einer Probe von genomischer DNA mit einem Reagens, das die unmethylierten Cytosinbasen in Uracil oder irgendeine andere Base, die zur Paarung mit einer anderen Base als Guanin fähig ist, umwandeln kann;
(ii) das Unterziehen eines Strangs der vorbehandelten genomischen DNA einer Amplifikation unter Verwendung mindestens zweier Oligonucleotide, wobei jedes mindestens 10 Nucleotide lang ist, als Amplifikationsprimer, wobei eines der Oligonucleotide zu einem ersten Segment einer Zielsequenz, die ausgewählt ist aus der Gruppe, bestehend aus SEQ ID NO: 1, SEQ ID NO: 2, SEQ ID NO: 5 und SEQ ID NO: 6, komplementär ist und das andere Oligonucleotid mit einem zweiten Segment dieser Zielsequenz identisch ist, wobei das zweite Segment stromabwärts vom ersten Segment gelegen ist und zumindest eines von den beiden Segmenten der Zielsequenz mindestens ein CpG-Dinucleotid umfasst; und
(iii) das Nachweisen des Vorhandenseins von amplifizierten Fragmenten der vorbehandelten genomischen DNA, wobei das Vorhandensein von amplifizierten Fragmenten auf eine neoplastische Erkrankung hinweist.

10. Verfahren gemäß Anspruch 7 oder Anspruch 8, umfassend die folgenden Schritte:
(i) das chemische Vorbehandeln einer Probe von genomischer DNA mit einem Reagens, das die unmethylierten Cytosinbasen in Uracil oder irgendeine andere Base, die zur Paarung mit einer anderen Base als Guanin fähig ist, umwandeln kann;
(ii) das Unterziehen eines Strangs der vorbehandelten genomischen DNA einer Amplifikation unter Verwendung mindestens zweier Oligonucleotide, wobei jedes mindestens 10 Nucleotide lang ist, als Amplifikationsprimer, wobei eines der Oligonucleotide zu einem ersten Segment einer Zielsequenz, die ausgewählt ist aus der Gruppe, bestehend aus SEQ ID NO: 1, SEQ ID NO: 2, SEQ ID NO: 5 und SEQ ID NO: 6, komplementär ist und das andere Oligonucleotid mit einem zweiten Segment dieser Zielsequenz identisch ist, wobei das zweite Segment stromabwärts vom ersten Segment gelegen ist; und
(iii) das Nachweisen des Vorhandenseins von amplifizierten Fragmenten der vorbehandelten genomischen DNA durch Hybridisierung mit einer Oligonucleotidsonde von mindestens 18 Nucleotiden Länge, die zu einem dritten Segment dieser Zielsequenz komplementär ist, wobei sich das dritte Segment zwischen dem ersten und dem zweiten Segment befindet und mindestens ein CpG-Dinucleotid umfasst, wobei das Vorhandensein von amplifizierten Fragmenten auf eine neoplastische Erkrankung hinweist.

11. Verfahren gemäß Anspruch 10, wobei die Sonde mit einem nachweisbaren Marker **gekennzeichnet ist**.

12. Verfahren gemäß einem der Ansprüche 9 bis 11, wobei die Amplifikation eine Polymerase-Kettenreaktion (PCR) ist.

13. Verfahren gemäß Anspruch 12, wobei die Amplifikation eine primäre PCR ist, gefolgt von einer halbverschachtelten oder verschachtelten sekundären PCR.

14. Verfahren gemäß einem der Ansprüche 9 bis 13, wobei es sich bei dem Reagens um Bisulfit, vorzugsweise Natriumbisulfit, in Verbindung mit Hydrochinon handelt.

15. Verfahren gemäß einem der Ansprüche 7 bis 14, wobei die neoplastische Erkrankung ein fester Tumor oder eine neoplastische Erkrankung des Blutes ist.

16. Verfahren gemäß Anspruch 15, wobei die neoplastische Erkrankung ausgewählt ist aus der Gruppe, bestehend aus primären Tumoren des Zentralnervensystems, zerebralen Metastasen, Tumoren des Auges, Tumoren der Hypophyse, Tumoren der Schilddrüse, Tumoren der Nebennierenrinde, Tumoren des diffusen endokrinen Systems, Tumoren des Kopfes und Halses, Tumoren der Lunge, bösartigem Mesotheliom, Thymomen und Thymustumoren, Tumoren des Herzens und großer Blutgefäße, primären thorakalen Keimzelltumoren, metastatischen Tumoren des Thorax, Speiseröhrentumoren, Lebertumoren, Magentumoren, Tumoren der Gallenblase und der Gallengänge, Tumoren des exokrinen Pankreas, Tumoren der Vater-Ampulle, Tumoren des Dünndarms, Tumoren des Wurmfortsatzes und Bauchfells, Tumoren des Dickdarms und Rektums, Tumoren des Anus, Nierentumoren, Tumoren des Beckens und Harnleiters, Tumoren der Blase, Tumoren der Prostata, Tumoren des Penis und der Harnröhre, testikulärem Tumor, Tumoren der Vulva und Vagina, Tumoren des Gebärmutterhalses, Gebärmutterschleimhautkrebs, Eileitertumoren, Eierstocktumoren, gynäkologischen Sarkomen, Brustdrüsentumoren, bösartigem Melanom, Tumoren der Haut, Tumoren des Knochens, Weichgewebesarkomen, Wilms-Tumoren, Neuroblastom, Rhabdomyosarkom, Kaposi-Sarkom, myelodysplastischem Syndrom, akuter myeloischer Leukämie beim Erwachsenen und beim Kind, chronischer myeloischer Leukämie, akuter Lymphozytenleukämie beim Erwachsenen, akuter lymphoblastischer Leukämie beim Kind, chronischer Lymphozytenleukämie, Haarzellenleukämie, Basophilenleukämie, Hodgkin-Krankheit, Nicht-Hodgkin-Lymphomen, Pilzmykose und Sézary-Syndrom, Plasmazelltumoren, chronischen myeloproliferativen Störungen.

17. Verfahren gemäß Anspruch 15, wobei die neoplastische Erkrankung ausgewählt ist aus der Gruppe, bestehend aus Magenkarzinom, kolorektalem Karzinom und Brustdrüsenkarzinom.

18. Kit für die Diagnose einer neoplastischen Erkrankung, umfassend ein Paar von Amplifikationsprimem, wie in Anspruch 9 oder Anspruch 10 definiert.

19. Kit gemäß Anspruch 18, zusätzlich umfassend eine Hybridisierungssonde, wie in Anspruch 10 definiert.

20. Kit gemäß Anspruch 18 oder Anspruch 19, zusätzlich umfassend ein Reagens, das die unmethylierten Cytosinbasen in Uracil oder irgendeine andere Base, die zur Paarung mit einer anderen Base als Guanin fähig ist, umwandeln kann.

21. Kit gemäß einem der Ansprüche 18 bis 20, zusätzlich umfassend ein zweites Paar von Amplifikationsprimern von mindestens 10 Nucleotiden Länge, wobei eines der Oligonucleotide zu einem ersten Segment einer Zielsequenz, die ausgewählt ist aus der Gruppe, bestehend aus SEQ ID NO: 3, SEQ ID NO: 4, SEQ ID NO: 7 und SEQ ID NO: 8, komplementär ist und das andere mit einem zweiten Segment dieser Zielsequenz identisch ist, wobei das zweite Segment stromabwärts vom ersten Segment gelegen ist.

22. Kit gemäß Anspruch 21, wobei zumindest eines von den beiden Segmenten der Zielsequenz mindestens ein TpG-Dinucleotid umfasst.

23. Kit gemäß einem der Ansprüche 18 bis 22, zusätzlich umfassend DNA-Amplifikationsmittel und/oder DNA-Nachweisreagenzien.

## Revendications

1. Acide nucléique comprenant une séquence nucléotidique choisie dans le groupe consistant en SEQ ID NO : 1, SEQ ID NO : 2, SEQ ID NO : 3, SEQ ID NO : 4, SEQ ID NO : 5, SEQ ID NO : 6, SEQ ID NO : 7 et SEQ ID NO : 8.

2. Oligonucléotide d'au moins 10 nucléotides de longueur, qui est complémentaire de, ou identique à, un segment d'une séquence cible choisie dans le groupe consistant en SEQ ID NO : 1, SEQ ID NO : 2, SEQ ID NO : 3, SEQ ID NO : 4, SEQ ID NO : 5, SEQ ID NO : 6, SEQ ID NO : 7 et SEQ ID NO : 8.

3. Oligonucléotide selon la revendication 2, d'au moins 18 nucléotides de longueur.

4. Oligonucléotide selon la revendication 2 ou la revendication 3, dans lequel ladite séquence cible est choisie dans le groupe consistant en SEQ ID NO : 1, SEQ ID NO : 2, SEQ ID NO : 5 et SEQ ID NO : 6, et ledit segment de la séquence cible comprend au moins un dinucléotide CpG.

5. Oligonucléotide selon la revendication 2 ou la revendication 3, dans lequel ladite séquence cible est choisie dans le groupe consistant en SEQ ID NO : 3, SEQ ID NO : 4, SEQ ID NO : 7 et SEQ ID NO : 8, et ledit segment de la séquence cible comprend au moins un dinucléotide TpG.

6. Oligonucléotide selon la revendication 1, choisi dans le groupe consistant en SEQ ID NO : 9, SEQ ID NO : 10, SEQ ID NO : 11, SEQ ID NO : 12, SEQ ID NO : 13, SEQ ID NO : 14 et SEQ ID NO : 15.

7. Procédé de diagnostic d'une maladie néoplasique comprenant l'étape d'analyse, dans un échantillon d'ADN génomique, de l'état de méthylation de la séquence nucléotidique du gène CDH4 entre les positions nucléotidiques correspondant aux nucléotides 46801 et 49600 de la séquence GenBank AL365229 (SEQ ID NO : 18), la méthylation de ladite séquence étant indicatrice d'une maladie néoplasique.

8. Procédé selon la revendication 7, comprenant l'étape d'analyse, dans un échantillon d'ADN génomique, de l'état de méthylation de la séquence nucléotidique du gène CDH4 entre les positions nucléotidiques correspondant aux nucléotides 47050 et 47376 de la séquence GenBank AL365229 (SEQ ID NO: 19), la méthylation de ladite séquence étant indicatrice d'une maladie néoplasique.

9. Procédé selon la revendication 7 ou la revendication 8, comprenant les étapes suivantes :
(i) pré-traitement chimique d'un échantillon d'ADN génomique avec un réactif capable de convertir les bases cytosines non méthylées en uracile ou en n'importe quelle autre base capable de s'apparier avec une base autre que la guanine ;
(ii) exposition d'un brin de l'ADN génomique prétraité à une amplification en utilisant en tant qu'amorces d'amplification au moins deux oligonucléotides, chacun d'au moins 10 nucléotides de longueur, un desdits oligonucléotides étant complémentaire d'un premier segment d'une séquence cible choisie dans le groupe consistant en SEQ ID NO : 1, SEQ ID NO : 2, SEQ ID NO : 5 et SEQ ID NO : 6 et l'autre oligonucléotide étant identique à un second segment de ladite séquence cible, le second segment étant en aval du premier segment, au moins un desdits premier et second segments de la séquence cible comprenant au moins un dinucléotide CpG ; et
(iii) détection de la présence de fragments amplifiés de l'ADN génomique prétraité, la présence de fragments amplifiés étant indicatrice d'une maladie néoplasique.

10. Procédé selon la revendication 7 ou la revendication 8, comprenant les étapes suivantes:
(i) pré-traitement chimique d'un échantillon d'ADN génomique avec un réactif capable de convertir les bases cytosines non méthylées en uracile ou en n'importe quelle autre base capable de s'apparier avec une base autre que la guanine ;
(ii) exposition d'un brin de l'ADN génomique prétraité à une amplification en utilisant en tant qu'amorces d'amplification au moins deux oligonucléotides, chacun d'au moins 10 nucléotides de longueur, un desdits oligonucléotides étant complémentaire d'un premier segment d'une séquence cible choisie dans le groupe consistant en SEQ ID NO : 1, SEQ ID NO : 2, SEQ ID NO : 5 et SEQ ID NO: 6 et l'autre oligonucléotide étant identique à un second segment de ladite séquence cible, le second segment étant en aval du premier segment ; et
(iii) détection de la présence de fragments amplifiés de l'ADN génomique prétraité par hybridation avec une sonde oligonucléotidique d'au moins 18 nucléotides de longueur qui est complémentaire d'un troisième segment de ladite séquence cible, ledit troisième segment étant situé entre ledit premier et ledit second segment et comprenant au moins un dinucléotide CpG, la présence de fragments amplifiés étant indicatrice d'une maladie néoplasique.

11. Procédé selon la revendication 10, dans lequel ladite sonde est étiquetée avec un marqueur détectable.

12. Procédé selon l'une quelconque des revendications 9 à 11, dans lequel ladite amplification est une amplification en chaîne par polymérase (Polymerase Chain Reaction ; PCR).

13. Procédé selon la revendication 12, dans lequel ladite amplification est une PCR primaire suivie par une PCR secondaire semi-nichée ou nichée.

14. Procédé selon l'une quelconque des revendications 9 à 13, dans lequel ledit réactif est un bisulfite, de préférence le bisulfite de sodium, en combinaison avec de l'hydroquinone.

15. Procédé selon l'une quelconque des revendications 7 à 14, dans lequel ladite maladie néoplasique est une tumeur solide ou une maladie néoplasique du sang.

16. Procédé selon la revendication 15, dans lequel ladite maladie néoplasique est choisie dans le groupe constitué des tumeurs primaires du système nerveux central, métastases cérébrales, tumeurs de l'oeil, tumeurs de l'hypophyse, tumeurs de la glande thyroïde, tumeurs de la corticosurrénale, tumeurs du système endocrinien diffus, tumeurs de la tête et du cou, tumeurs des poumons, mésothéliomes malins, thymomes et tumeurs thymiques, tumeurs du coeur et des grands vaisseaux, tumeurs des cellules germinales thoraciques primaires, tumeurs métastatiques du thorax, tumeurs oesophagiennes, tumeurs du foie, tumeurs gastriques, tumeurs de la vésicule biliaire et des canaux biliaires, tumeurs du pancréas exocrine, tumeurs de l'ampoule de Vater, tumeurs de l'intestin grêle, tumeurs de l'appendice et du péritoine, tumeurs du côlon et du rectum, tumeurs de l'anus, tumeurs rénales, tumeurs du pelvis et de l'uretère, tumeurs de la vessie, tumeurs de la prostate, tumeurs du pénis et de l'urètre, tumeurs testiculaires, tumeurs de la vulve et du vagin, tumeurs du col, cancers de l'endomètre, tumeurs des tubes de Fallope, tumeurs ovariennes, sarcomes gynécologiques, tumeurs mammaires, mélanomes malins, tumeurs de la peau, tumeurs de l'os, sarcomes des tissus mous, de la tumeur de Wilms, des neuroblastomes, des rhabdomyosarcomes, du sarcome de Kaposi, des syndromes myélodysplasiques, de la leucémie myéloïde aiguë de l'adulte et de l'enfant, leucémie myéloïde chronique, leucémie aiguë lymphocytaire de l'adulte, leucémie aiguë lymphoblastique de l'enfant, leucémie lymphocytaire chronique, leucémie à tricholeucocytes, leucémie à mastocytes, maladie de Hodgkin, des lymphomes non hodgkiniens, du mycosis fongoïde et du syndrome de Sézary, des tumeurs des cellules plasmatiques, des syndromes myéloprolifératifs chroniques.

17. Procédé selon la revendication 15, dans lequel ladite maladie néoplasique est choisie dans le groupe constitué du cancer de l'estomac, du cancer du côlon et du rectum et du cancer du sein.

18. Kit de diagnostic d'une maladie néoplasique, comprenant une paire d'amorces d'amplification telles que définies à la revendication 9 ou à la revendication 10.

19. Kit selon la revendication 18, comprenant de plus une sonde d'hybridation telle que définie à la revendication 10.

20. Kit selon la revendication 18 ou la revendication 19, comprenant de plus un réactif capable de convertir les bases cytosines non méthylées en uracile ou en n'importe quelle autre base capable de s'apparier avec une base autre que la guanine.

21. Kit selon l'une quelconque des revendications 18 à 20, comprenant de plus une seconde paire d'amorces d'amplification d'au moins 10 nucléotides de longueur, un desdits oligonucléotides étant complémentaire d'un premier segment d'une séquence cible choisie dans le groupe consistant en SEQ ID NO : 3, SEQ ID NO : 4, SEQ ID NO : 7 et SEQ ID NO : 8 et l'autre étant identique à un second segment de ladite séquence cible, le second segment étant en aval du premier segment.

22. Kit selon la revendication 21, dans lequel au moins un desdits premier et second segments de la séquence cible comprend au moins un dinucléotide TpG.

23. Kit selon l'une quelconque des revendications 18 à 22, comprenant de plus un moyen d'amplification de l'ADN et/ou des réactifs de détection de l'ADN.


### SEQUENCE LISTING

<110> Università degli Studi di Ferrara
   <120> An in vitro method for the diagnosis of neoplastic diseases by methylation analysis of the CDH4 gene, oligonucleotides and kits useful in such method
   <130>
   <160> 19
   <170> PatentIn version 3.1
<210> 1
   <211> 2800
   <212> DNA
   <213> Artificial Sequence
   <220>
   <223> modified artificial sequence obtained from completely methylated CpG island
<400> 1
210> 2
   <211> 2800
   <212> DNA
   <213> Artificial Sequence
   <220>
   <223> modified artificial sequence obtained from completely methylated CpG island
   <400> 2
<210> 3
   <211> 2800
   <212> DNA
   <213> Artificial Sequence
   <220>
   <223> modified artificial sequence obtained from non-methylated CpG island
   <400> 3
   agtgtatatt tttattgtgg gtttgggtgg gaagttggat taagattgtg gtgggattgt 60
   tttatttaga aagggtgttt tgggtgtggg ggtggaaatt ttagtgttgt ttttagtttt 120
   tatatttgtg tttttttttt ttggaaaatg agtgtttttg gtttttttgg ttgtaattgg 180
   gtggttttag atgtttagga gggtagaggt tgggttggtg tggtgtgggg ggtgtgggtg 240
   tgggttttgt gtttttaagg tttgtgttgg tagagtgagt gtaggggttg ggtggtgggg 300
   tggggttttg gtggggtggt tgtggtttta tttgtagttt tgagtgtgtg gaatagaggt 360
   gtgggtggtt gtgaggttgg tggatgtatt ggtttgaggg aatgtgttgt tggttggatt 420
   tggtttggtt tttggagttg gggtggtgag tgtggtgggt gtagtggggt tggaggtttt 480
   gggtaggtgt gggggagtgg tggtggtggt ggtgattgga gtggtggtgg tggttttggt 540
   ggtggtggtg gtggtggtgg tagggagtgg gtttttggtg ttgggtattg ggtgggtggt 600
   ggggaagatg attgtgggtg ttggtgtgtt ttttttgttg tttttgtttt ttggtgtgtt 660
   ttgggtaagt tgttgttttt tgtttttgtt gtttggaagt tttgggtagt gggaggttgt 720
   ttttggattt tgtggggtgt ttatatattt ggtggggttt ttttgggttt ttttgttgtg 780
   ttttttgttg tatttagttt ggtgtttgtg tttggtgaag ttgtttgtgg ttggtaggag 840
   tgggaagttg tttgggtagt ggggagtggt ggagtagggt gggagtgggg tttggtggag 900
   gatttgggga gttttgtttg tattggattt gttgagtttt ttttgaatgt ttaaagtttg 960
   tagttgttat ttggagtttg gtttgtttgt atggggtagg tttttattgg tggagttggt 1020
   gtgtttttta ttgtttggga gttgtgtgtt gtgggagttg ttgagtttag ttttggttgt 1080
   ggttgaaggt gtggggtgtt tggtggttgt gggttaggga tttgtgagtg tggggtgttt 1140
   gtttgatttt ttttgtttaa ggtttggggg tttgtttagt ggtgttgttt agttttgtgg 1200
   gtgttggtag gatttgtgtt gtgggtgggt ggtggggtag gattgtgggg tatttttatt 1260
   tttgagtttt tggtggggtt ggagtttgtg tgtttggtgg ggttttgggg gttgggagtt 1320
   gggttgggtt tgggttggga agttggatgt tgggtgggtg tggtgtggga gggtgttttg 1380
   ggagggtgag tgggtttttt gttgtttttg tagagttttg tgggttggat tgttagtttt 1440
   gttttgtgtt tggtgaggtg tgtggtttgt gggggtagag agtgtggtgg ttttgggggt 1500
   gtttttgggt ggaggtggag gggtggtggg agttgtagga attttttttt gtggttgatt 1560
   ggttttgttg tttttttttg ggtagtgttg ttgtaatttt ggaaataaat aagtgttagt 1620
   ggtgggaggt ttgttttgag gttgtggggt gttgggggat gtgttgggta tatgttttgg 1680
   gtgggtggaa atttttttgg gtgagttgag ttttttattg ggttgtgggt gtgatttgga 1740
   gttttgtggt tgatgaagaa agttgtggtt tagagttttt tgtaaggaat gattttgtgt 1800
   tttgtttttt ttttttggtg ttgggggtag aggggtgttt tttgtggtgt tggtgttggg 1860
   aagtttggag ttttgggttg ggggtggttt ttggagttga agtatgggtg ggtgatggta 1920
   gtgtgtggag ttggaggagg tggtgggtgg gaggtgttat tgtttggtgt gggttgtatt 1980
   tgaggttttt tgtgtttgtt ggggagttaa gtattgttgt gtttttgttt ttttagtggg 2040
   gttggttttt tgtttgtata tattgttttt ttttttatta tttttttttg tttgttatgt 2100
   ttttttttag aagtttggtt ggagggtttt gttgtttatt ttattttttt ttggttttgt 2160
   gttatgggtt ttagattttt ttattgagta ggaaaggtga gtgagttttg gaggatagga 2220
   ggagattttt tttttggtta gttaggggag ttgttttttt gtttgggttg gagatggggg 2280
   ttttgagggt gaaaatgtgg gttgaagatt agaaggtgga agtgttttta ttgttttttt   2340
   attttagtta ttggtagttt tttgtagagt tgtttttttt gattggtttt ggatttagtt   2400
   ttagagaatt tttttttttt tttttttttt tttttttttt tttggtgatt tggagaggag   2460
   ataagaagtt gttttttttg ttttattaag agtgtagagt ggtgggttta aagtgtgaat   2520
   ggtttgagta gagttgttta tattttggtt tttttgtttt tagtggtttt gggagtagtg   2580
   gttgaagttt tttgtttttg ttttttggtt attttttagg tgtttttttt gttaataagg   2640
   ttttggttgg tttggttttt ttaatagttt gttttttatt ttttgtttgt tgtgtgtggg   2700
   gttggggaag gatgtttttg gtttgtagtt tggagagtgt tttttaaagt ttttagagag   2760
   tagaggagta gatggggtat tgtgtttggg tggagatggt                         2800
<210> 4
   <211> 2800
   <212> DNA
   <213> Artificial Sequence
   <220>
   <223> modified artificial sequence obtained from non-methylated CpG island
   <400> 4
   attgttttta tttagatgtg atattttgtt tgtttttttg ttttttgggg attttaggga     60
   gtatttttta ggttatagat taggagtatt tttttttagt tttatatata gtaggtaggg    120
   agtgaaggat ggattgttgg ggaagttagg ttggttaggg ttttattggt agaggagatg    180
   tttgagaaat gattaaaagg tagagataga agattttagt tgttgttttt gaagttatta    240
   agggtaggga ggttaaagtg tgggtggttt tgtttagatt atttgtgttt taggtttatt    300
   gttttgtgtt tttggtaagg tggaggaggt agttttttgt ttttttttta ggttattggg    360
   agggaggaga aagaggagag aaaggaagaa gattttttag gattaggttt ggagttggtt    420
   gaagaaggta gttttgtggg aggttgttgg tggttggggt gaggaggtag tgggggtgtt    480
   tttatttttt agtttttagt ttgtattttt atttttggag tttttatttt tagtttaggt    540
   agggagatgg tttttttaat tgattggaaa gggggttttt ttttgttttt tggagtttgt    600
   ttgttttttt tgtttagtgg ggaagtttag agtttatggt atagggttag ggaaggatgg    660
   ggtggatgat agggtttttt gattaagttt ttgggagggg gtgtgatagg taggaggagg    720
   tggtggaggg agaggtagtg tgtgtaagtg ggaagttagt tttgttgaag gagtaggagt    780
   atagtggtgt ttggtttttt ggtgagtgta ggaagttttg ggtgtagttt gtattgggta    840
   gtggtgtttt ttgtttgttg ttttttttag ttttgtgtgt tgttgttgtt tgtttatgtt    900
   ttgattttgg aggttgtttt tagtttggag ttttaggttt tttgatgttg gtattgtgga    960
   gggtgttttt ttatttttag tattgaggga ggagagtggg atgtagggtt attttttatg   1020
   aaaagtttta aattgtggtt ttttttattg gttatgaagt tttaaattgt atttgtagtt   1080
   tggtgggagg tttaatttgt ttaggaaagt ttttgtttgt ttgaagtgtg tgtttagtgt   1140
   gttttttagt gttttgtggt tttggagtgg gttttttgtt gttggtattt gtttattttt   1200
   gaggttataa tagtgttgtt tgggagaagg tagtgaggtt gattggttat gagagggggt   1260
   ttttgtaatt tttgttgttt ttttgttttt gtttaggggt gtttttggag ttgttgtgtt   1320
   ttttgttttt gtgggttgtg tattttgtta ggtgtagagt aaagttggta atttggtttg   1380
   tggggttttg tagaggtagt gggagatttg tttgtttttt tggggtgttt ttttgtgttg   1440
   tgtttgtttg gtgtttggtt ttttggttta agtttggttt agtttttagt ttttggggtt   1500
   ttgttggata tgtaggtttt agttttgttg agggtttggg ggtgagagtg ttttgtagtt   1560
   ttattttgtt gtttatttgt agtgtgggtt ttgttggtat ttatagaatt gagtggtgtt   1620
   gttagatgga tttttaggtt ttgggtagga gaggttgggt aggtgttttg tatttgtgga   1680
   tttttggttt gtagttgttg ggtgttttat gtttttagtt atggttgggg ttgggtttgg   1740
   taatttttgt ggtgtgtggt ttttgggtga tggagggtgt gttggttttg ttagtggaag   1800
   tttgttttat gtaggtaagt tgagttttgg gtagtggtta tgggttttgg gtgtttgagg   1860
   gaggtttggt gggtttggtg taggtggagt tttttgggtt ttttattgag ttttattttt   1920
   attttgtttt gttgtttttt gttgtttagg tggttttttg tttttgttga ttgtaggtag   1980
   ttttgttgag tgtgggtgtt gggttggatg tagtggggag tgtggtgggg gagtttggga   2040
   gagttttgtt gggtgtgtga gtgttttgtg ggatttgggg atgatttttt gttgtttggg   2100
   gtttttgaat ggtgggggtg ggaggtggta atttatttgg agtgtgttgg agagtgagag   2160
   tagtagaagg agtatgttgg tgtttgtggt tatttttttt gttgtttgtt tggtgtttgg   2220
   tattgggagt ttgttttttg ttgttgttgt tgttgttgtt gttgagatta ttgttgttgt   2280
   tttgattgtt gttgttgttg ttgttttttt gtgtttgttt ggagttttta gttttgttgt   2340
   gtttgttgtg tttgttgttt tggttttggg agttaggtta agtttggttg gtggtgtgtt   2400
   tttttgggtt ggtgtgtttg ttggttttgt agttgtttgt gtttttgttt tgtgtgtttg   2460
   gagttgtagg tgaagttgtg gttgttttgt tgaggttttg ttttgttgtt tggtttttgt   2520
   gtttgttttg ttgatgtggg ttttaaaggt gtagagtttg tgtttgtgtt ttttgtgttg   2580
   tattagttta gtttttgttt ttttggatgt ttggagttgt ttggttgtgg ttgaagaggt   2640
   taaggatgtt tgttttttag gggaaaaagg tgtgggtgtg gagattgaag atagtgttgg   2700
   ggtttttgtt tttatatttg gggtattttt tttggatggg gtggttttgt tgtggtttta   2760
   gtttgatttt ttgtttgggt ttgtggtggg agtgtgtatt                         2800
<210> 5
   <211> 327
   <212> DNA
   <213> Artificial Sequence
   <220>
   <223> sequence comprised between nucleotides 250-576 of SEQ ID NO:1
   <400> 5
   cgtttttaag gttcgcgtcg gtagagcgag cgtaggggtc gggcggcggg gcggggtttt     60
   ggcggggcgg tcgcggtttt atttgtagtt tcgagcgcgc ggaatagagg cgcgggcggt    120
   tgcgaggtcg gcggacgtat cggttcgagg gaacgcgtcg tcggtcggat ttggtttggt    180
   tttcggagtc ggggcggcga gcgcggcggg cgtagcgggg ttggaggttt cgggtaggcg    240
   cgggggagcg gcggcggcgg cggcgatcgg agcggcggcg gtggtttcgg cggcggcggt    300
   ggcggcggcg gtagggagcg ggttttt                                        327
<210> 6
   <211> 327
   <212> DNA
   <213> Artificial Sequence
   <220>
   <223> sequence comprised between nucleotides 2225-2551 of SEQ ID NO:2
   <400> 6
   gggagttcgt tttttgtcgt cgtcgtcgtc gtcgtcgtcg agattatcgt cgtcgtttcg      60
   atcgtcgtcg tcgtcgtcgt tttttcgcgt ttgttcggag tttttagttt cgttgcgttt     120
   gtcgcgttcg tcgtttcggt ttcgggagtt aggttaagtt cggtcggcgg cgcgtttttt     180
   cgggtcggtg cgttcgtcgg tttcgtagtc gttcgcgttt ttgtttcgcg cgttcggagt     240
   tgtaggtgaa gtcgcggtcg tttcgtcgag gtttcgtttc gtcgttcggt ttttgcgttt     300
   gttttgtcga cgcgggtttt aaaggcg                                         327
<210> 7
   <211> 327
   <212> DNA
   <213> Artificial Sequence
   <220>
   <223> sequence comprised between nucleotides 250-576 of SEQ ID NO:3
   <400> 7
   tgtttttaag gtttgtgttg gtagagtgag tgtaggggtt gggtggtggg gtggggtttt      60
   ggtggggtgg ttgtggtttt atttgtagtt ttgagtgtgt ggaatagagg tgtgggtggt     120
   tgtgaggttg gtggatgtat tggtttgagg gaatgtgttg ttggttggat ttggtttggt     180
   ttttggagtt ggggtggtga gtgtggtggg tgtagtgggg ttggaggttt tgggtaggtg     240
   tgggggagtg gtggtggtgg tggtgattgg agtggtggtg gtggttttgg tggtggtggt     300
   ggtggtggtg gtagggagtg ggttttt                                         327
<210> 8
   <211> 327
   <212> DNA
   <213> Artificial Sequence
   <220>
   <223> sequence comprised between nucleotides 2225-2551 of SEQ ID NO:4
   <400> 8
   gggagtttgt tttttgttgt tgttgttgtt gttgttgttg agattattgt tgttgttttg      60
   attgttgttg ttgttgttgt ttttttgtgt ttgtttggag tttttagttt tgttgtgttt     120
   gttgtgtttg ttgttttggt tttgggagtt aggttaagtt tggttggtgg tgtgtttttt     180
   tgggttggtg tgtttgttgg ttttgtagtt gtttgtgttt ttgttttgtg tgtttggagt     240
   tgtaggtgaa gttgtggttg ttttgttgag gttttgtttt gttgtttggt ttttgtgttt     300
   gttttgttga tgtgggtttt aaaggtg                                         327
<210> 9
   <211> 20
   <212> DNA
   <213> Artificial Sequence
   <220>
   <223> cdh4_MF
<400> 9
   cgtttttaag gttcgcgtcg 20
<210> 10
   <211> 20
   <212> DNA
   <213> Artificial Sequence
   <220>
   <223> cdh4_M2F
   <400> 10
   tcggcgttaa cgcgttcggc 20
<210> 11
   <211> 19
   <212> DNA
   <213> Artificial Sequence
   <220>
   <223> cdh4_M3F
   <400> 11
   tttgtagttt cgagcgcgc 19
<210> 12
   <211> 18
   <212> DNA
   <213> Artificial Sequence
   <220>
   <223> cdh4_MR
   <400> 12
   gaaaacccgc tccctacc 18
<210> 13
   <211> 23
   <212> DNA
   <213> Artificial Sequence
   <220>
   <223> cdh4_M2R
   <400> 13
   ataaaaacga cctcgcgacg cgc 23
<210> 14
   <211> 22
   <212> DNA
   <213> Artificial Sequence
   <220>
   <223> cdh4_UF
   <400> 14
   gtgtttttaa ggtttgtgtt gg 22
<210> 15
   <211> 18
   <212> DNA
   <213> Artificial Sequence
   <220>
   <223> cdh4_UR
   <400> 15
   aaaaacccac tccctacc 18
<210> 16
   <211> 20
   <212> DNA
   <213> Artificial Sequence
   <220>
   <223> cdh4_249F
   <400> 16
   gttggggcag atgggacagt 20
<210> 17
   <211> 20
   <212> DNA
   <213> Artificial Sequence
   <220>
   <223> cdh4_547R
   <400> 17
   gttggggcag atgggacagt 20
<210> 18
   <211> 2800
   <212> DNA
   <213> Homo sapiens
   <400> 18
<210> 19
   <211> 327
   <212> DNA
   <213> Homo sapiens
   <400> 19
   cgcctttaag gcccgcgtcg gcagagcgag cgcaggggcc gggcggcggg gcggggcctc     60
   ggcggggcgg ccgcggcttc acctgcagct ccgagcgcgc ggaacagagg cgcgggcggc    120
   tgcgaggccg gcggacgcac cggcccgagg gaacgcgccg ccggccggac ttggcctggc    180
   tcccggagcc ggggcggcga gcgcggcggg cgcagcgggg ctggaggctc cgggcaggcg    240
   cgggggagcg gcggcggcgg cggcgatcgg agcggcggcg gtggtctcgg cggcggcggc    300
   ggcggcggcg gcagggagcg ggctccc                                        327

### SEQUENCE LISTING

<110> Università degli Studi di Ferrara
   <120> An in vitro method for the diagnosis of neoplastic diseases by methylation analysis of the CDH4 gene, oligonucleotides and kits useful in such method
   <130>
   <160> 19
   <170> PatentIn version 3.1
<210> 1
   <211> 2800
   <212> DNA
   <213> Artificial Sequence
   <220>
   <223> modified artificial sequence obtained from completely methylated CpG island
<400> 1
   agtgtatatt tttatcgcgg gttcgggcgg gaagtcggat taagatcgcg gcgggatcgt      60
   tttatttaga aagggtgttt cgggtgtggg ggcggaaatt ttagcgttgt ttttagtttt     120
   tatattcgcg tttttttttt ttggaaaacg agcgtttttg gttttttcgg tcgtaatcgg    180
   gcggttttag acgtttagga gggtagaggt tgggttggtg cggcgcgggg ggcgcgggcg     240
   cgggttttgc gtttttaagg ttcgcgtcgg tagagcgagc gtaggggtcg ggcggcgggg     300
   cggggtttcg gcggggcggt cgcggtttta tttgtagttt cgagcgcgcg gaatagaggt     360
   gcgggcggtt gcgaggtcgg cggacgtatc ggttcgaggg aacgcgtcgt cggtcggatt     420
   tggtttggtt ttcggagtcg gggcggcgag cgcggcgggc gtagcggggt tggaggtttt     480
   gggtaggcgc gggggagcgg cggcggcggc ggcgatcgga gcggcggcgg tggtttcggt     540
   ggcggcggcg gcggcggcgg tagggagcgg gttttcggtg tcgggtatcg ggcgggcggt     600
   ggggaagatg atcgcgggcg tcggcgtgtt ttttttgttg ttttcgtttt tcggcgcgtt     660
   tcgggtaagt tgtcgttttt cgttttcgtc gttcggaagt ttcgggtagc gggaggtcgt     720
   tttcggattt cgcggggcgt ttatatattc ggcggggttt tttcgggttt tttcgtcgcg     780
   tttttcgttg tatttagttc ggcgttcgcg ttcggcgaag ttgtttgcgg tcggtaggag     840
   cgggaagtcg tttgggtagc ggggagcggc ggagtagggt gggagtgggg ttcggtggag     900
   gattcgggga gtttcgtttg tatcggattc gtcgagtttt tttcgaacgt ttaaagttcg     960
   tagtcgttat tcggagttcg gtttgtttgt atggggtagg tttttattgg cggagtcggt    1020
   gcgtttttta tcgttcggga gtcgcgcgtc gcgggagttg tcgagtttag tttcggtcgt    1080
   ggttgaaggc gtggggcgtt cggcggttgc gggttaggga ttcgcgagtg cggggcgttt    1140
   gttcgatttt ttttgtttaa ggtttggggg ttcgtttagc ggcgtcgttt agttttgtgg    1200
   gtgtcggtag gattcgcgtt gcgggtgggc ggcggggtag gattgcgggg tatttttatt    1260
   ttcgagtttt cggcggggtt ggagtttgcg tgttcggcgg ggtttcgggg gttgggagtt    1320
   gggtcgggtt tgggtcggga agtcggacgt cgggcgggcg cggcgcggga gggcgtttcg    1380
   ggagggcgag cgggtttttc gttgtttttg tagagtttcg cgggtcggat tgttagtttt   1440
   gttttgcgtt tggcgaggtg cgcggttcgc gggggtagag agcgcggcgg tttcgggggt   1500
   gtttttgggc ggaggcggag gggcggcggg agttgtagga attttttttc gtggtcgatt  1560
   ggtttcgttg ttttttttcg ggtagcgttg ttgtaatttc ggaaataaat aagtgttagt   1620
   ggcgggaggt tcgtttcgag gtcgcggggc gttgggggac gcgttgggta tacgtttcgg   1680
   gcgggcggaa atttttttgg gcgagttgag ttttttatcg ggttgcgggt gcgatttgga  1740
   gtttcgtggt cgatgaagaa agtcgcggtt tagagttttt cgtaaggaat gattttgcgt   1800
   ttcgtttttt tttttcggtg ttgggggtag aggggcgttt ttcgcggtgt cggcgtcggg   1860
   aagtttggag tttcgggttg ggggcggttt tcggagtcga agtatgggcg ggcgacggta   1920
   gcgcgcggag ttggaggagg cggcgggcgg gaggcgttat tgttcggtgc gggttgtatt   1980
   cgaggttttt tgcgttcgtc ggggagttaa gtatcgttgt gtttttgttt ttttagcggg   2040
   gttggttttt cgtttgtata tattgttttt ttttttatta tttttttttg tttgttacgt  2100
   ttttttttag aagtttggtc ggagggtttt gtcgtttatt ttattttttt ttggttttgt   2160
   gttatgggtt ttagattttt ttattgagta ggaaaggcga gcgagtttcg gaggatagga   2220
   ggagattttt ttttcggtta gttaggggag tcgttttttt gtttgggttg gagatggggg   2280
   tttcgagggt gaaaatgcgg gttgaagatt agaaggtgga agcgttttta ttgttttttt   2340
   attttagtta tcggtagttt ttcgtagagt tgtttttttc gatcggtttc ggatttagtt   2400
   ttagagaatt tttttttttt tttttttttt tttttttttt ttcggtgatt tggagaggag   2460
   ataagaagtt gtttttttcg ttttattaag agcgtagagc ggtgggttta aagcgcgaat  2520
   ggtttgagta gagtcgttta tattttggtt tttttgtttt tagtggtttc gggagtagcg   2580
   gttgaagttt tttgtttttg ttttttggtt attttttagg cgtttttttt gttaataagg 2640
   ttttggtcgg tttggttttt ttaatagttc gttttttatt ttttgtttgt tgtgtgtggg   2700
   gttggggaag gatgtttttg gtttgtagtt tggagagtgt tttttaaagt ttttagagag   2760
   tagaggagta gacggggtat cgcgtttggg tggagacggt                         2800
<210> 2
   <211> 2800
   <212> DNA
   <213> Artificial Sequence
   <220>
   <223> modified artificial sequence obtained from completely methylated CpG island
   <400> 2 <210> 3
   <211> 2800
   <212> DNA
   <213> Artificial Sequence
   <220>
   <223> modified artificial sequence obtained from non-methylated CpG island <400> 3
<210> 4
   <211> 2800
   <212> DNA
   <213> Artificial Sequence
   <220>
   <223> modified artificial sequence obtained from non-methylated CpG island <400> 4
<210> 5
   <211> 327
   <212> DNA
   <213> Artificial Sequence
   <220>
   <223> sequence comprised between nucleotides 250-576 of SEQ ID NO:1
   <400> 5
   cgtttttaag gttcgcgtcg gtagagcgag cgtaggggtc gggcggcggg gcggggtttt 60
   ggcggggcgg tcgcggtttt atttgtagtt tcgagcgcgc ggaatagagg cgcgggcggt 120
   tgcgaggtcg gcggacgtat cggttcgagg gaacgcgtcg tcggtcggat ttggtttggt 180
   tttcggagtc ggggcggcga gcgcggcggg cgtagcgggg ttggaggttt cgggtaggcg 240
   cgggggagcg gcggcggcgg cggcgatcgg agcggcggcg gtggtttcgg cggcggcggt 300
   ggcggcggcg gtagggagcg ggttttt 327
<210> 6
   <211> 327
   <212> DNA
   <213> Artificial Sequence
   <220>
   <223> sequence comprised between nucleotides 2225-2551 of SEQ ID NO:2
   <400> 6
   gggagttcgt tttttgtcgt cgtcgtcgtc gtcgtcgtcg agattatcgt cgtcgtttcg     60
   atcgtcgtcg tcgtcgtcgt tttttcgcgt ttgttcggag tttttagttt cgttgcgttt    120
   gtcgcgttcg tcgtttcggt ttcgggagtt aggttaagtt cggtcggcgg cgcgtttttt    180
   cgggtcggtg cgttcgtcgg tttcgtagtc gttcgcgttt ttgtttcgcg cgttcggagt    240
   tgtaggtgaa gtcgcggtcg tttcgtcgag gtttcgtttc gtcgttcggt ttttgcgttt    300
   gttttgtcga cgcgggtttt aaaggcg                                         327
<210> 7
   <211> 327
   <212> DNA
   <213> Artificial Sequence
   <220>
   <223> sequence comprised between nucleotides 250-576 of SEQ ID NO:3
   <400> 7
   tgtttttaag gtttgtgttg gtagagtgag tgtaggggtt gggtggtggg gtggggtttt     60
   ggtggggtgg ttgtggtttt atttgtagtt ttgagtgtgt ggaatagagg tgtgggtggt    120
   tgtgaggttg gtggatgtat tggtttgagg gaatgtgttg ttggttggat ttggtttggt    180
   ttttggagtt ggggtggtga gtgtggtggg tgtagtgggg ttggaggttt tgggtaggtg    240
   tgggggagtg gtggtggtgg tggtgattgg agtggtggtg gtggttttgg tggtggtggt    300
   ggtggtggtg gtagggagtg ggttttt                                         327
<210> 8
   <211> 327
   <212> DNA
   <213> Artificial Sequence
   <220>
   <223> sequence comprised between nucleotides 2225-2551 of SEQ ID NO:4
   <400> 8
   gggagtttgt tttttgttgt tgttgttgtt gttgttgttg agattattgt tgttgttttg     60
   attgttgttg ttgttgttgt ttttttgtgt ttgtttggag tttttagttt tgttgtgttt    120
   gttgtgtttg ttgttttggt tttgggagtt aggttaagtt tggttggtgg tgtgtttttt    180
   tgggttggtg tgtttgttgg ttttgtagtt gtttgtgttt ttgttttgtg tgtttggagt    240
   tgtaggtgaa gttgtggttg ttttgttgag gttttgtttt gttgtttggt ttttgtgttt    300
   gttttgttga tgtgggtttt aaaggtg                                         327
<210> 9
   <211> 20
   <212> DNA
   <213> Artificial Sequence
<220>
   <223> cdh4_MF
   <400> 9
   cgtttttaag gttcgcgtcg 20
<210> 10
   <211> 20
   <212> DNA
   <213> Artificial Sequence
   <220>
   <223> cdh4_M2F
   <400> 10
   tcggcgttaa cgcgttcggc 20
<210> 11
   <211> 19
   <212> DNA
   <213> Artificial Sequence
   <220>
   <223> cdh4_M3F
   <400> 11
   tttgtagttt cgagcgcgc 19
<210> 12
   <211> 18
   <212> DNA
   <213> Artificial Sequence
   <220>
   <223> cdh4_MR
   <400> 12
   gaaaacccgc tccctacc 18
<210> 13
   <211> 23
   <212> DNA
   <213> Artificial Sequence
   <220>
   <223> cdh4_M2R
   <400> 13
   ataaaaacga cctcgcgacg cgc 23
<210> 14
   <211> 22
   <212> DNA
   <213> Artificial Sequence
   <220>
   <223> cdh4_UF
   <400> 14
   gtgtttttaa ggtttgtgtt gg 22
<210> 15
   <211> 18
   <212> DNA
   <213> Artificial Sequence
   <220>
   <223> cdh4_UR
   <400> 15
   aaaaacccac tccctacc 18
<210> 16
   <211> 20
   <212> DNA
   <213> Artificial Sequence
   <220>
   <223> cdh4_249F
   <400> 16
   gttggggcag atgggacagt 20
<210> 17
   <211> 20
   <212> DNA
   <213> Artificial Sequence
   <220>
   <223> cdh4_547R
   <400> 17
   gttggggcag atgggacagt 20
<210> 18
   <211> 2800
   <212> DNA
   <213> Homo sapiens
   <400> 18
   agtgcacact cccaccgcgg gcccgggcgg gaagtcggac taagaccgcg gcgggaccgc 60
   cccatccaga aagggtgccc cgggtgtggg ggcggaaacc ccagcgctgt cttcagtctc    120
   cacacccgcg cctttttccc ctggaaaacg agcgtccttg gcctcttcgg ccgcaaccgg    180
   gcggctccag acgtccagga gggcagaggc tgggctggtg cggcgcgggg ggcgcgggcg    240
   cgggctctgc gcctttaagg cccgcgtcgg cagagcgagc gcaggggccg ggcggcgggg    300
   cggggcctcg gcggggcggc cgcggcttca cctgcagctc cgagcgcgcg gaacagaggc    360
   gcgggcggct gcgaggccgg cggacgcacc ggcccgaggg aacgcgccgc cggccggact    420
   tggcctggct cccggagccg gggcggcgag cgcggcgggc gcagcggggc tggaggctcc    480
   gggcaggcgc gggggagcgg cggcggcggc ggcgatcgga gcggcggcgg tggtctcggc    540
   ggcggcggcg gcggcggcgg cagggagcgg gctcccggtg ccgggcaccg ggcgggcggc    600
   ggggaagatg accgcgggcg ccggcgtgct ccttctgctg ctctcgctct ccggcgcgct    660
   ccgggtaagt tgccgcctcc cgcccccgcc gttcggaagc cccgggcagc gggaggtcgt    720
   ccccggatcc cgcggggcgc tcacacaccc ggcggggctc tcccgggctc ccccgccgcg    780
   ctccccgctg catccagccc ggcgcccgcg ctcggcgaag ctgcctgcgg tcggcaggag    840
   cgggaagccg cctgggcagc ggggagcggc ggagcagggt gggagtgggg ctcggtggag    900
   gacccgggga gctccgcctg caccggaccc gccgagcctc cctcgaacgc ccaaagcccg    960
   tagccgctac ccggagctcg gcttgcctgc atggggcagg cttccactgg cggagccggc   1020
   gcgccctcca tcgcccggga gccgcgcgcc gcgggagttg ccgagcccag ccccggccgt   1080
   ggctgaaggc gtggggcgcc cggcggctgc gggccaggga tccgcgagtg cggggcgcct   1140
   gcccgacctc tcctgcccaa ggcctggggg tccgtctagc ggcgccgctc agttctgtgg   1200
   gtgccggcag gacccgcgct gcgggtgggc ggcggggtag gactgcgggg cactctcacc   1260
   cccgagccct cggcggggct ggagcctgcg tgtccggcgg ggccccgggg gctgggagct   1320
   gggccgggct tgggccggga agccggacgc cgggcgggcg cggcgcggga gggcgccccg   1380
   ggagggcgag cgggtctccc gctgcctctg cagagccccg cgggccggat tgccagcttt   1440
   gctctgcgcc tggcgaggtg cgcggcccgc gggggcagag agcgcggcgg ctccgggggc   1500
   gcccctgggc ggaggcggag gggcggcggg agttgcagga accccctctc gtggccgatc   1560
   ggcctcgctg ccttctcccg ggcagcgctg ttgtaacctc ggaaataaac aagtgccagc   1620
   ggcgggaggc ccgctccgag gccgcggggc gctgggggac gcgctgggca cacgcttcgg   1680
   gcgggcggaa actttcctgg gcgagttgag cctcccaccg ggctgcgggt gcgatttgga   1740
   gcttcgtggc cgatgaagaa agccgcggtt tagagctttt cgtaaggaat gaccctgcgt   1800
   cccgctctcc tccctcggtg ctgggggtag aggggcgccc tccgcggtgc cggcgtcggg   1860
   aagcctggag ctccgggctg ggggcggcct ccggagtcga agcatgggcg ggcgacggca   1920
   gcgcgcggag ctggaggagg cggcgggcgg gaggcgccac tgcccggtgc gggctgcacc   1980
   cgaggcttcc tgcgctcgcc ggggagccaa gcaccgctgt gctcctgctc cttcagcggg   2040
   gctggcttcc cgcttgcaca cactgcctct ccctccacca cctcctcctg cctgtcacgc   2100
   cccctcccag aagcttggtc ggagggccct gtcgtccacc ccatccttcc ctggccctgt   2160
   gccatgggct ctagacttcc ccactgagca ggaaaggcga gcgagctccg gaggacagga   2220
   ggagaccccc tttccggtca gttaggggag ccgtctccct gcctgggctg gagatggggg   2280
   ctccgagggt gaaaatgcgg gctgaagact agaaggtgga agcgccccca ctgcctcctc   2340
   accccagcca ccggcagcct cccgcagagc tgccttcttc gaccggctcc ggacctagtc   2400
   ctagagaatc ttcttccttt ctctcctctt tctcctccct cccggtgacc tggagaggag   2460
   acaagaagct gcctcctccg ccttaccaag agcgcagagc ggtgggccta aagcgcgaat   2520
   ggtctgagca gagccgccca cactttggcc tccctgccct tagtggcttc gggagcagcg   2580
   gctgaagtct tctgtctctg ccttttggtc atttctcagg cgtctcctct gccaataagg   2640
   ccctggccgg cctggcttcc ccaacagtcc gtccttcact ccctgcctgc tgtgtgtggg   2700
   gctggggaag gatgctcctg gtctgtagcc tggagagtgc tccctaaagt ccccagagag   2760
   cagaggagca gacggggtat cgcgtctggg tggagacggt                         2800
<210> 19
   <211> 327
   <212> DNA
   <213> Homo sapiens
   <400> 19
   cgcctttaag gcccgcgtcg gcagagcgag cgcaggggcc gggcggcggg gcggggcctc     60
   ggcggggcgg ccgcggcttc acctgcagct ccgagcgcgc ggaacagagg cgcgggcggc    120
   tgcgaggccg gcggacgcac cggcccgagg gaacgcgccg ccggccggac ttggcctggc    180
   tcccggagcc ggggcggcga gcgcggcggg cgcagcgggg ctggaggctc cgggcaggcg    240
   cgggggagcg gcggcggcgg cggcgatcgg agcggcggcg gtggtctcgg cggcggcggc    300
   ggcggcggcg gcagggagcg ggctccc                                        327